# EUROPEAN PATENT APPLICATION

(11) **EP 4 151 264 A1**
(43) Date of publication of application: **22.03.2023**
(21) Application number: 21804762.9
(22) Date of filing: 16.04.2021
(51) Int. Cl.: A61M 25/00, A61B 17/22, A61B 17/32

(54) **CATHETER AND CATHETER SYSTEM**

(30) Priority: 15.05.2020 JP 2020086022
(71) Applicant: Asahi Intecc Co., Ltd., Seto-shi, Aichi 489-0071 (JP)
(72) Inventor: NAMIMA, Satoshi, Seto-shi, Aichi 489-0071 (JP); OSHIMA, Fumiyoshi, Seto-shi, Aichi 489-0071 (JP); KUBO, Yuta, Seto-shi, Aichi 489-0071 (JP)
(74) Representative: Winter, Brandl - Partnerschaft mbB
(86) International application number: PCT/JP2021/015653
(87) International publication number: WO 2021/229999

(57) **Abstract**

A catheter A having a lumen 14 includes a tube 10, a sensor unit 25 disposed at a distal end of the tube 10 and capable of transmitting and receiving ultrasound, and an irradiation unit 26 disposed at the distal end of the tube 10 and capable of emitting the ultrasound toward a guide wire 15 which passes through the lumen 14 and protrudes from the distal end of the tube 10. The shape of the biological tissue can be observed by transmitting the ultrasound from the sensor units 25 toward the biological tissue and receiving the reflection wave reflected by the biological tissue at the sensor units 25. Irradiating the guide wire 15 with the ultrasound transmitted from the irradiation unit 26 causes the guide wire 15 to vibrate. The vibration of the guide wire 15 can facilitate the passage through an occlusion site of the biological tissue.

## Description

### TECHNICAL FIELD

The disclosed embodiments relate to a catheter and a catheter system.

### BACKGROUND

Patent literature 1 discloses a medical device in which a guide wire is inserted into a catheter and a "spring" is provided as a vibration generating means. A distal end portion of the guide wire protruding from a distal end portion of the catheter is caused to vibrate by the "spring", thereby allowing passage through the vascular occlusion.

### CITATION LIST

### Patent Literature

Patent Literature 1: JP 2013-518690 A

### SUMMARY

### TECHNICAL PROBLEM

The above-mentioned medical device has a function of facilitating the passage through the vascular occlusion by the guide wire, but does not have a function of detecting the shape of the vascular occlusion site. Before performing the procedure of passing through the vascular occlusion by vibrating the guide wire, the shape of the vascular occlusion site needs to be diagnosed by using another catheter. This requires a device for observing the shape of the vascular occlusion site in addition to the medical device for passing through the vascular occlusion site.

The disclosed embodiments have been completed based on the above-mentioned circumstances and have an object of providing a catheter capable of exerting a function of observing the shape of a biological tissue and a function of facilitating the passage through an occlusion site of the biological tissue. In particular, in a case where the occlusion site formed in the biological tissue is hardened due to calcification or the like, thereby making it difficult for a guide wire to pass through the occlusion site, the disclosed embodiments have an object of making it easier for the guide wire to crush and pass through the hardened occlusion site.

### SOLUTION TO PROBLEM

(1) The present disclosure provides a catheter including: a tube having a lumen; a sensor unit disposed at a distal end of the tube and capable of transmitting and receiving ultrasound; and an irradiation unit disposed at the distal end of the tube and capable of emitting the ultrasound toward a medical device that passes through the lumen and protrudes from the distal end of the tube,
(2) the catheter described in the above-mentioned (1) in which the irradiation unit is configured by arranging a plurality of oscillators in a row and the medical device is disposed on a line extended in an arrangement direction of the plurality of oscillators,
(3) the catheter described in the above-mentioned (2) in which the irradiation unit includes a first transmission unit and a second transmission unit disposed so as to sandwich the lumen and the plurality of oscillators constituting the first transmission unit and the second transmission unit are disposed to be aligned on a straight line,
(4) the catheter described in any of the above-mentioned (1) to (3) in which the irradiation unit is configured by including the oscillators that can be controlled independently of the oscillators constituting the sensor unit,
(5) the catheter described in any of the above-mentioned (1) to (4) in which the oscillators constituting the sensor unit and the oscillators constituting the irradiation unit are composed of the same kind of component,
(6) the catheter described in any of the above-mentioned (1) to (5) in which the sensor unit is configured by including the oscillators capable of transmitting the ultrasound toward the medical device,
(7) the catheter described in the above-mentioned (6) in which the sensor unit has a mode of emitting the ultrasound toward a lesion and a mode of emitting the ultrasound toward the medical device, in the mode of emitting the ultrasound toward the lesion, the sensor unit being controlled to switch between transmission and reception of the ultrasound in each predetermined period, in the mode of emitting the ultrasound toward the medical device, the sensor unit being controlled to continuously transmit the ultrasound for a period longer than the predetermined period,
(8) the catheter described in any of the above-mentioned (1) to (7) in which the irradiation unit is configured by including the oscillators capable of transmitting and receiving the ultrasound,
(9) the catheter described in the above-mentioned (8) in which, in the mode of transmitting and receiving the ultrasound, the irradiation unit is configured to perform the transmission and reception of the ultrasound intermittently and alternately, and
(10) a catheter system including an emission control unit that is connected to the catheter described in any of the above-mentioned (1) to (9) and that controls ultrasound transmitting of the sensor unit and the irradiation unit, in which the emission control unit controls display on a display device on the basis of the ultrasound received by the sensor unit.

### ADVANTAGEOUS EFFECTS

The shape of the biological tissue can be observed by transmitting the ultrasound from the sensor unit toward the biological tissue and receiving a reflection wave reflected by the biological tissue at the sensor unit. When the medical device is irradiated with the ultrasound transmitted from the irradiation unit, the medical device is caused to vibrate, and the vibration of the medical device can facilitate the passage through the occlusion site of the biological tissue. The catheter of the disclosed embodiments can exert a function of observing the shape of the biological tissue and a function of facilitating the passage through the occlusion site of the biological tissue.

### BRIEF DESCRIPTION OF DRAWINGS

FIG. 1 is a side cross-sectional view of a catheter system of a first embodiment.
FIG. 2 is an elevation view of a catheter.
FIG. 3 is a side cross-sectional view of the catheter.
FIG. 4 is a side cross-sectional view of the catheter into which a medical device is inserted.
FIG. 5 is an elevation view of a catheter of a second embodiment.
FIG. 6 is an elevation view of a catheter of a third embodiment.
FIG. 7 is an elevation view of a catheter of a fourth embodiment.
FIG. 8 is an elevation view of a catheter of a fifth embodiment.

### EMBODIMENTS

In the present embodiment, an irradiation unit may be configured by arranging a plurality of oscillators in a row, and a medical device may be disposed on a line extended in an arrangement direction of the plurality of oscillators. According to this configuration, when the timing of ultrasound transmission is shifted relative to one another among the plurality of oscillators, a plurality of ultrasound wavefronts are combined, so that ultrasound beams can be converged and focused on the medical device. This can cause the medical device to strongly vibrate, thereby facilitating passage through an occlusion site of a biological tissue.

In the present embodiment, the irradiation unit may include a first transmission unit and a second transmission unit disposed so as to sandwich a lumen, and the plurality of oscillators constituting the first transmission unit and the second transmission unit may be arranged to be aligned on a straight line. According to this configuration, the medical device is irradiated with two converged ultrasound beams and thus can be more strongly vibrated.

In the present embodiment, the irradiation unit may be configured by including the oscillators that can be controlled independently of the oscillators constituting the sensor unit. According to this configuration, it becomes possible to observe the biological tissue by the sensor unit and facilitate the passage through the occlusion site of the biological tissue by the irradiation unit at the same time.

In the present embodiment, the oscillators constituting the sensor unit and the oscillators constituting the irradiation unit may be composed of the same kind of component. According to this configuration, it becomes possible to reduce costs as only one kind of oscillator is required to constitute the sensor unit and the irradiation unit.

In the present embodiment, the sensor unit may have a mode of emitting the ultrasound toward the biological tissue and a mode of emitting the ultrasound toward the medical device. In the mode of emitting the ultrasound toward the biological tissue, the sensor unit may be controlled to switch between transmission and reception of the ultrasound in each predetermined period. In the mode of emitting the ultrasound toward the medical device, the sensor unit may be controlled to continuously transmit the ultrasound for a period longer than the predetermined period. According to this configuration, it becomes possible to strongly vibrate the medical device by using both the irradiation unit and the sensor unit after observing the biological tissue.

In the present embodiment, the irradiation unit may be configured by including the oscillators capable of transmitting and receiving the ultrasound. According to this configuration, it becomes possible to observe the biological tissue by the sensor unit and the irradiation unit before performing the passage through the occlusion site of the biological tissue. In this configuration, the number of the transmission position and the reception position of the ultrasound is increased as compared with the case where the observation is performed only by the sensor unit, thus observation accuracy of the shape of the biological tissue improves.

In the present embodiment, in the mode in which the irradiation unit transmits and receives the ultrasound, the transmission and reception of the ultrasound may be performed intermittently and alternately. According to this configuration, the observation of the biological tissue can be surely performed by the irradiation unit.

In the embodiments described below, a mode in which a medical device such as a guide wire is passed through a lumen of the catheter and disposed in front of the irradiation unit will be described. According to this configuration, the medical device is passed through the lumen of the catheter and disposed in front of the irradiation unit, thereby exhibiting an effect of facilitating the focus of the ultrasound emitted from the irradiation unit on the medical device.

Further, the medical device may be disposed in front of the irradiation unit so as to face a front of the catheter in the longitudinal direction or by passing by the side of the catheter. When the medical device is disposed in front of the sensor unit and the irradiation unit so as to face the front of the catheter in the longitudinal direction or by passing by the side of the catheter, it becomes possible to place the catheter and the medical device in separate biological tissues and facilitate the passage through the occlusion site of the biological tissue different from the one in which the catheter is placed. The configuration of the disclosed embodiments having the sensor unit and the irradiation unit at the distal end portion of the catheter makes it possible to perform the transmission and reception of the ultrasound according to various purposes such as observation of the position of the biological tissue and the medical device and facilitation of the passage through the occlusion site.

### <First embodiment>

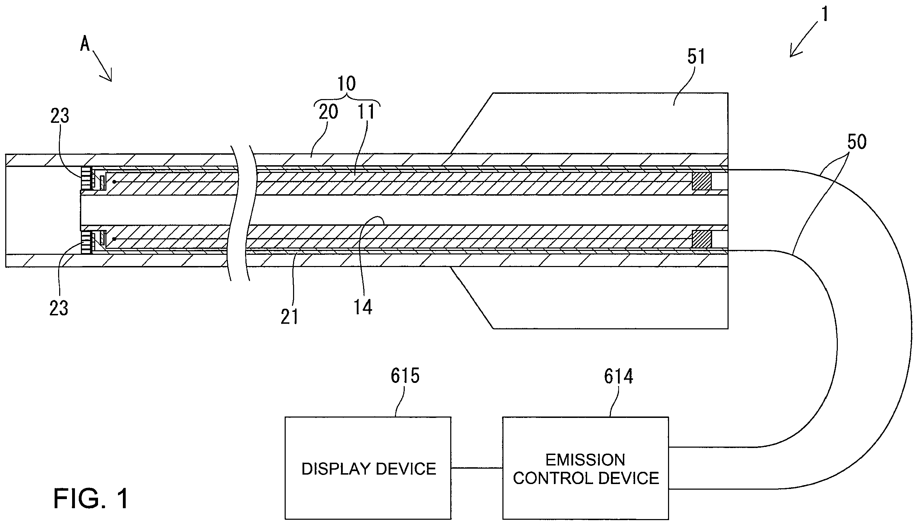

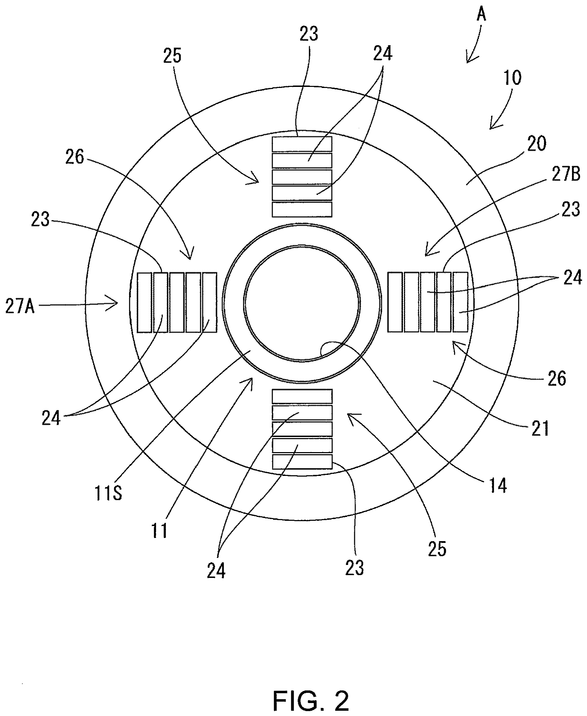

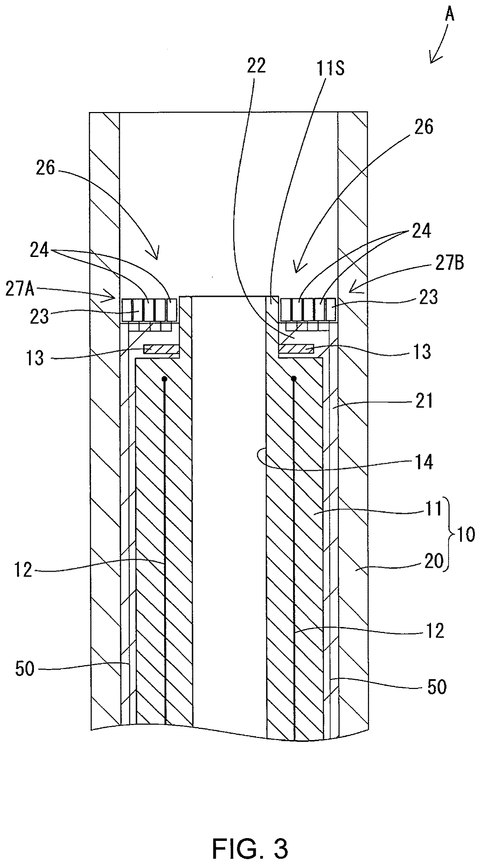

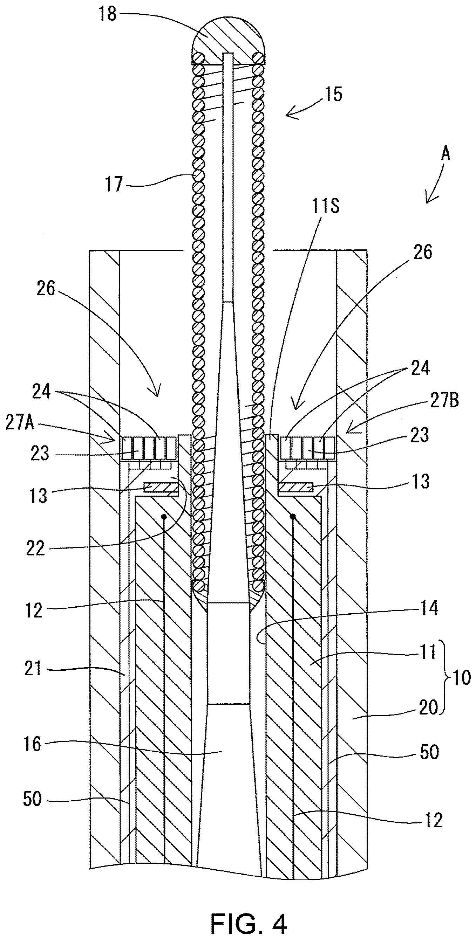

Hereinafter, a first embodiment embodying the disclosed embodiments will be described with reference to FIG. 1 to FIG. 4. FIG. 1 shows a catheter system 1 including a catheter A and an emission control device 614 and FIG. 2 shows an elevation view of the catheter A viewed from the front. The catheter system 1 is not limited to the first embodiment and has the similar configurations in second to fifth embodiments. In the following description, regarding a front-back direction, an upper side in FIG. 3 and FIG. 4 is defined as a front side. The terms "front end" and "distal end" of the catheter A are used with the same meaning.

The catheter A and the catheter system 1 of the first embodiment include one flexible tube 10, a lumen 14 formed inside the tube 10, four ultrasound sensors 23, an emission control device 614 for controlling the ultrasound sensors 23, a display device 615 for displaying information acquired from the ultrasound sensor 23, and a connector 51 for connecting the ultrasound sensors 23 and the emission control device 614 using signal lines 50 and for facilitating an operation of inserting the catheter A into the body.

FIG. 2 shows an elevation view of the catheter A. The tube 10 has a configuration in which a long inner tube 11 having a circular cross section is coaxially housed in a long outer tube 20 having a long circular cross section in a state that restricts relative displacement in the radial direction. A flexible guide wire 15 is inserted through the lumen 14 of the catheter A.

FIG. 3 shows a cross-sectional view of the catheter A on a distal end side with the guide wire 15 not being inserted therein and FIG. 4 shows a cross-sectional view of the catheter A on the distal end side with the guide wire 15 being inserted therein. As shown in FIG. 3 and FIG. 4, pull wires 12 are embedded in the inner tube 11. The pull wires 12 are used for changing a direction of a distal end portion of the catheter A. Further, the inner tube 11 and the outer tube 20 can be rotated relatively to each other in the circumferential direction by performing a rotational operation of the inner tube 11 while holding the outer tube 20 at a proximal end of the catheter A, for example, by rotating the connector 51.

On the outer peripheral surface of the inner tube 11, a support tube 21 having a cylindrical shape concentric with the inner tube 11 and the outer tube 20 is provided so as to move integrally with the inner tube 11. A support portion 22 concentrically protruding to the inner peripheral side is formed at the distal end of the support tube 21. The support portion 22 fits into a small diameter portion 11S on the outer periphery of the inner tube 11 at the distal end and is located slightly behind the distal end of the inner tube 11. The distal end of the inner tube 11 (a front end portion of the small diameter portion 11S) is protruded frontward from a central hole of the support portion 22. The inner peripheral surface of the support portion 22 is in close contact with the outer peripheral surface of the small diameter portion 11S. The outer peripheral surface of the support tube 21 is formed of a member having low friction such as PTFE. The outer peripheral surface of the support tube 21 and the inner peripheral surface of the outer tube 20 slide against each other, allowing the outer tube 20, and the support tube 21 and the inner tube 11 to rotate relatively to each other.

Note that, as a different mode of the first embodiment, the inner peripheral surface of the support tube 21 may be formed of PTFE or the like, so that the inner peripheral surface of the support tube 21 and the outer peripheral surface of the inner tube slide against each other, allowing the outer tube 20 and the inner tube 11 to rotate relatively to each other.

A pressure sensor 13 is equipped inside the support portion 22. The pressure sensor 13 is connected to a wire (not shown) routed inside the inner tube 11. The pressure sensor 13 detects that the distal end of the tube 10 collides with the biological tissue (not shown). Note that the present embodiment describes the mode in which the pressure sensor 13 detects that the distal end of the tube 10 collides with the biological tissue. However, the collision with the biological tissue may be detected by using ultrasound sensors 23 described below without including the pressure sensor 13.

The space inside the inner tube 11 forms the lumen 14. The lumen 14 has a circular cross-sectional shape. The guide wire 15 as a medical device is inserted in the lumen 14. The guide wire 15 includes a core wire 16, a coil body 17 formed by a wire or a stranded wire, and a distal tip 18. The coil body 17 is disposed so as to surround a front end portion of the core wire 16, and a rear end portion of the coil body 17 is fixed to the core wire 16 by brazing. The distal tip 18 is fixed to a front end portion of the coil body 17 by brazing.

The guide wire 15 can be relatively displaced in the longitudinal direction in the lumen 14. When the guide wire 15 is displaced forward, that is, toward the distal end side, the distal tip 18 protrudes more forward than the front end of the tube 10. When the guide wire 15 is displaced toward the proximal end side, the distal tip 18 is housed inside the tube 10. The distal tip 18 is caused to vibrate, while the coil body 17 is elastically deformed, by irradiation with the ultrasound.

The four ultrasound sensors 23 are attached to a front surface of the support portion 22. The signal lines 50 connecting the ultrasound sensors 23 and the emission control device 614 are inserted or embedded inside the support tube 21. As shown in FIG. 2, when the catheter A is viewed, as an elevation view, from the distal end side in the longitudinal direction, the four ultrasound sensors 23 are concentrically disposed around the lumen 14 with being spaced at equal angular intervals in the circumferential direction. Each ultrasound sensor 23 is configured by including a plurality of oscillators 24 arranged in a row in the radial direction. The plurality of oscillators 24 are radially arranged around the lumen 14.

Each oscillator 24 is composed of a piezoelectric element using a piezoelectric material such as lead zirconate titanate (PZT). As the oscillator 24, a polymer such as PVDF (polyvinylidene fluoride) or a MEMS device such as CMUT may be used in addition to PZT. The oscillator 24 has a transmission function of transmitting the ultrasound to the front of the catheter A by applying a voltage and a reception function of generating a voltage by receiving the ultrasound from the front of the catheter A.

In the first embodiment, two of the four ultrasound sensors 23 are used as sensor units 25 for observing the biological tissue. The oscillators 24 constituting the sensor units 25 are connected to the emission control device 614. The emission control device 614 has a circuit (not shown) that applies a voltage for transmitting the ultrasound to the oscillators 24 and a circuit (not shown) that generates an ultrasound image on the basis of a voltage generated by receiving the ultrasound. Further, the circuit that generates the ultrasound image is connected to a display device 615. The two ultrasound sensors 23, which function as the sensor units 25, are disposed so as to sandwich the lumen 14. The plurality of oscillators 24 constituting the two ultrasound sensors 23 that function as the sensor units 25 are disposed to be aligned on a straight line with the lumen 14 intervening between them.

Of the four ultrasound sensors 23, two ultrasound sensors 23 other than the sensor units 25 are used as irradiation units 26 for emitting the ultrasound to the distal tip 18. The oscillators 24 are connected to the circuit that applies the voltage for transmitting the ultrasound to the oscillators 24, but not connected to the circuit that generates the ultrasound image on the basis of the voltage generated by receiving the ultrasound.

The two ultrasound sensors 23 that function as the irradiation units 26 constitute a first transmission unit 27A and a second transmission unit 27B. The plurality of oscillators 24 constituting the first transmission unit 27A and the second transmission unit 27B are disposed to be aligned on a straight line with the lumen 14 intervening between them. In an elevation view, a direction (up-down direction in FIG. 2) in which the plurality of oscillators 24 functioning as the sensor units 25 are aligned and a direction (right-left direction in FIG. 2) in which the plurality of oscillators 24 functioning as the irradiation units 26 are aligned perpendicularly intersect each other. That is, the oscillators 24 of the sensor units 25 and the oscillators 24 of the irradiation units 26 are disposed in a cross shape so as to intersect each other at the center of the lumen 14.

As a drive system of the plurality of oscillators 24 constituting each sensor unit 25, it is possible to adopt a phased array system in which a transmission voltage is applied to each oscillator constituting the plurality of oscillators 24 at slightly different timing. Secondary wavefronts generated by a plurality of ultrasound waves transmitted at different timing form focused ultrasound with high energy. Like the oscillators 24 constituting the sensor units 25, a driving voltage may be applied to the plurality of oscillators 24 constituting each irradiation unit 26 at slightly different timing.

A focus position where the ultrasound transmitted from each of the two sensor units 25 disposed across the lumen 14 is converged can be adjusted by changing the transmission timing of the ultrasound among the plurality of oscillators 24 constituting each sensor unit 25. When the time difference of the ultrasound transmission is reduced, a distance from the front end of catheter A to the focus position becomes longer. The focus position is adjusted to the lumen 14 side of the sensor units 25 by transmitting the ultrasound from the oscillators 24 on the outer peripheral side before the oscillators 24 on the lumen 14 side among the plurality of oscillators 24 arranged in a row in the radial direction. This makes it possible to adjust the focus position toward the distal tip 18 side of the guide wire 15 that passes through and protrudes from the lumen 14 by controlling the transmission time difference and the transmission order among the plurality of oscillators 24 of each sensor unit 25 when the ultrasound is transmitted from the two sensor units 25 across the lumen 14.

A usage mode of the catheter A of the first embodiment will be described. The purpose is to eliminate an occlusion site formed in a tubular biological tissue such as a blood vessel and improve the circulation of the blood or the like. When a biological tissue is observed, the ultrasound is intermittently transmitted each for a short period of time from the oscillators 24 of the pair of sensor units 25 in a state where the guide wire 15 is housed in the tube 10. The pulse-like ultrasound transmitted from the sensor units 25 forms the focused ultrasound and irradiates the biological tissue, and then reflected on the surface or at the inside of the biological tissue. A reflection wave reflected by the biological tissue is received by the oscillators 24 in a transmission suspended state. The oscillators 24 that receive the ultrasound generate a voltage according to the intensity of the reflection wave. An ultrasound image (not shown) of the biological tissue is obtained on the basis of the voltage generated by the oscillators 24. The biological tissue is observed in this manner.

When the observation of the biological tissue is completed, the guide wire 15 is displaced forward, the distal tip 18 is protruded more forward than the tube 10 and pressed against the occlusion site formed in the biological tissue, and the distal end of the outer tube 20 is pressed against the occlusion site or the biological tissue near the occlusion site. Whether or not the outer tube 20 is in contact with the occlusion site or the biological tissue near the occlusion site is detected by the pressure sensors 13 or the time between the transmission and reception of the ultrasound using the oscillators 24. The ultrasound is transmitted from the oscillators 24 of the pair of irradiation units 26 with the distal tip 18 being pressed against the occlusion site. The outer tube 20 being pressed against the occlusion site or the biological tissue near the occlusion site allows the catheter A to hold the guide wire 15 when the distal tip 18 of the guide wire 15 is vibrated, making it possible to efficiently transmit the vibration of the distal tip 18 to the occlusion site.

The focus position of the transmitted ultrasound is set so that the ultrasound is converged at the distal tip 18. The distal tip 18 vibrates at a high frequency by being irradiated with the focused ultrasound having high energy. In this process, if the frequency of the focused ultrasound is made the same as the natural frequency of the distal tip 18, it becomes possible to amplify the amplitude of the distal tip 18 by resonating the distal tip 18. The vibrating distal tip 18 facilitates crushing and passing through the occlusion site. As a specific example, the distal tip 18 can improve the blood flow by destroying the occlusion site in the blood vessel inner wall and allowing the guide wire 15 to pass through the occlusion site.

The irradiation units 26 are configured by including the oscillators 24 that are controlled independently of the oscillators 24 constituting the sensor units 25. That is, the sensor units 25 can freely transmit and receive the ultrasound without being restricted by the ultrasound transmission of the irradiation units 26. Thus, it is possible to perform observation of the biological tissue by the sensor units 25 and facilitation of the passage through the occlusion site by the irradiation units 26 at the same time.

The catheter A of the first embodiment includes the tube 10 having the lumen 14, the sensor units 25, and the irradiation units 26. The sensor units 25 are disposed at the distal end of the tube 10 and capable of transmitting and receiving the ultrasound. The shape of the biological tissue can be observed by transmitting the ultrasound from the sensor units 25 toward the biological tissue and receiving the reflection wave reflected by the biological tissue at the sensor units 25. The irradiation units 26 are disposed at the distal end of the tube 10 and can emit the ultrasound toward the guide wire 14 passing through the lumen 14 and projecting further toward the distal end direction from the distal end of the tube 10. When the ultrasound transmitted from the irradiation units 26 is emitted to the distal tip 18 of the guide wire 15, the distal tip 18 is caused to vibrate and, by pressing the vibrating distal tip 18 against the occlusion site, the occlusion site can be easily passed through. The catheter A of the first embodiment can exert the function of observing the shape of the biological tissue and the function of facilitating the passage through the occlusion site of the biological tissue.

The irradiation units 26 are configured by arranging the plurality of oscillators 24 in a row, and the lumen 14 through which the guide wire 15 is inserted is disposed on a line extended in an arrangement direction of the plurality of oscillators 24. The first transmission unit 27A and the second transmission unit 27B constituting the irradiation units 26 are disposed so as to sandwich the lumen 14. The plurality of oscillators 24 constituting the first transmission unit 27A and the plurality of oscillators 24 constituting the second transmission unit 27B are disposed so as to be aligned on a straight line. According to this configuration, when the timing of the ultrasound transmission is shifted among the plurality of oscillators 24, a plurality of ultrasound wavefronts are combined, so that ultrasound beams can be converged and focused on the distal tip 18 of the guide wire 15. Moreover, since the distal tip 18 is irradiated with the two converged ultrasound beams, the distal tip 18 can be vibrated more strongly. This can facilitate the passage through the occlusion site.

Since the oscillators 24 constituting the sensor units 25 and the oscillators 24 constituting the irradiation units 26 are composed of the same kind of component, only one kind of the oscillator 24 is required to constitute the sensor units 25 and the irradiation units 26. Thus, it becomes possible to reduce costs of the catheter A.

### <Second embodiment>

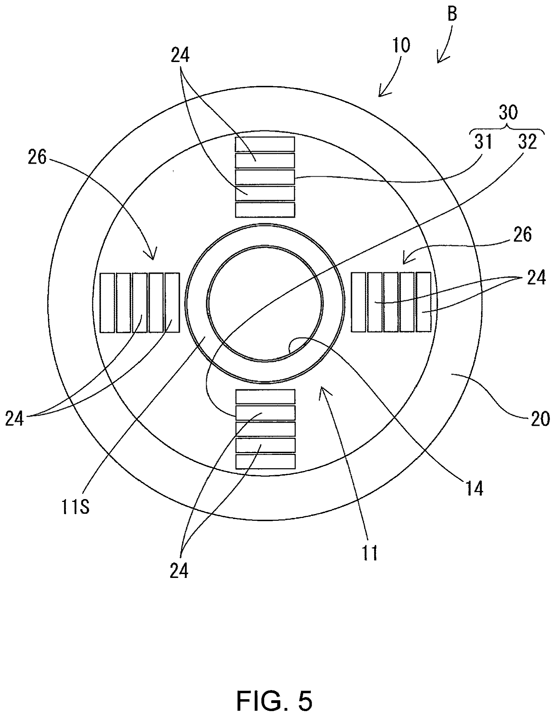

Next, a second embodiment embodying the disclosed embodiments will be described with reference to FIG. 5. In a catheter B of the second embodiment, a sensor unit 30 has a configuration different from that in the above-mentioned first embodiment. Other configurations are the same as those in the above-mentioned first embodiment, thus the same configurations are designated by the same reference numerals, and description of the structure, functions, and effects thereof is omitted.

The sensor unit 30 of the second embodiment is configured by including one transmission unit 31 and one reception unit 32 disposed at different positions from each other. The transmission unit 31, the reception unit 32, and the two irradiation units 26 are, as in the first embodiment, disposed to be aligned at an equal angular pitch in the circumferential direction around the lumen 14 and the guide wire 15 which is a separate body from the catheter B, not shown in FIG. 5. The transmission unit 31 and the reception unit 32 are disposed so as to sandwich the lumen 14 and the guide wire 15. The plurality of oscillators 24 constituting the transmission unit 31 are arranged in a row in the radial direction. The plurality of oscillators 24 constituting the reception unit 32 are also arranged in a row in the radial direction.

The oscillators 24 constituting the transmission unit 31 and the oscillators 24 constituting the reception unit 32 are piezoelectric elements having a function of transmitting the ultrasound by applying a voltage and a function of generating a voltage by receiving the ultrasound. The oscillators 24 constituting the transmission unit 31 are connected to the emission control device 614. The emission control device 614 is connected to a circuit (not shown) that applies a voltage for transmitting the ultrasound to the oscillators 24, but not connected to a circuit (not shown) that generates an ultrasound image on the basis of a voltage generated by receiving the ultrasound. Thus, the oscillators 24 of the transmission unit 31 are used as dedicated components that only transmit the ultrasound.

The oscillators 24 constituting the reception unit 32 is connected to the circuit that generates the ultrasound image on the basis of the voltage generated by receiving the ultrasound, but not connected to the circuit that applies the voltage for transmitting the ultrasound to the oscillators 24. Thus, the oscillators 24 of the reception unit 32 are used as dedicated components that only receive the ultrasound.

When the shape of the biological tissue is observed, the transmission unit 31 can continuously transmit the ultrasound and the reception unit 32 can continuously receive the reflection wave reflected by the biological tissue. In this manner, information received by the reception unit 32 is not interrupted, making it possible to easily recognize a change in the position of the occlusion site formed in the biological tissue and the guide wire 15 using the ultrasound image. Further, when the irradiation units 26 transmit the ultrasound while the sensor units 30 transmit and receive the ultrasound, it becomes possible to perform the observation of the biological tissue and the crushing of the occlusion site formed in the biological tissue at the same time.

### <Third embodiment>

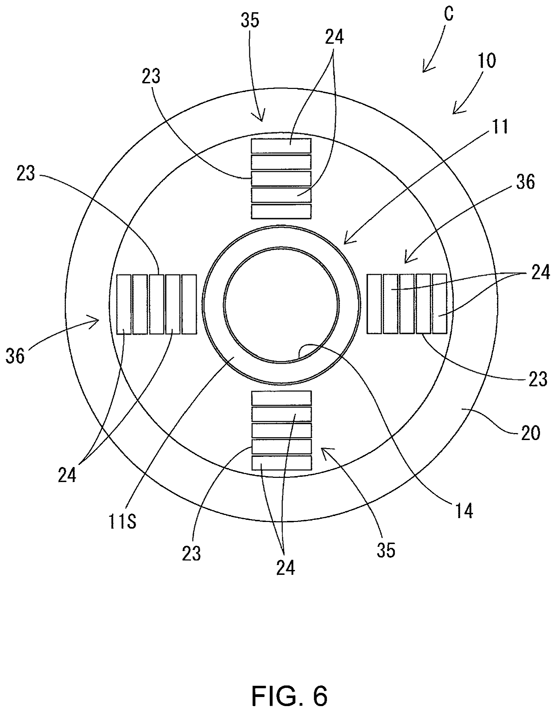

Next, a third embodiment embodying the disclosed embodiments will be described with reference to FIG. 6. In a catheter C of the third embodiment, a sensor unit 35 and an irradiation unit 36 have a configuration different from that in the above-mentioned first embodiment. Other configurations are the same as those in the above-mentioned first embodiment, thus the same configurations are designated by the same reference numerals, and description of the structure, functions, and effects thereof is omitted.

The catheter C of the third embodiment includes four ultrasound sensors 23. The ultrasound sensors 23 are, as in the first embodiment and the second embodiment, disposed to be aligned at an equal angular pitch in the circumferential direction around the lumen 14 and the guide wire 15 which is a separate body from the catheter C, not shown in FIG. 6. The four ultrasound sensors 23 include the oscillators 24 of the same kind. Each ultrasound sensor 23 is, as in the first and second embodiments, configured by the plurality of oscillators 24 arranged in a row in the radial direction. All of the oscillators 24 constituting the four ultrasound sensors 23 are piezoelectric elements of the same kind.

All of the oscillators 24 constituting the four ultrasound sensors 23 are connected to the emission control device 614. The emission control device 614 is connected to a circuit (not shown) that applies a voltage for transmitting the ultrasound to the oscillators 24 and a circuit (not shown) that generates an ultrasound image on the basis of a voltage generated by receiving the ultrasound. All of the four ultrasound sensors 23 have both a transmission function of transmitting the ultrasound to the front of the catheter C by applying a voltage and a reception function of generating a voltage by receiving the ultrasound from the front of the catheter C.

Thus, all of the ultrasound sensors 23 are controlled so as to switch between the transmission and reception of the ultrasound in each predetermined period. By this control, the ultrasound sensors 23 have both a function as the sensor unit 35 that observes the shape of biological tissue located in front of the catheter C and a function as the irradiation unit 36 that emits the ultrasound toward the distal tip 18 not shown in FIG. 6 for crushing the occlusion site of the biological tissue located in front of the catheter C.

A first usage mode of the catheter C will be described. Some (for example, two) of the four ultrasound sensors 23 are set as the sensor units 35 and the remaining ultrasound sensors 23 are set as the irradiation units 36. In a case where the observation of the biological tissue and the crushing of the occlusion site are performed in separate steps, the ultrasound is first transmitted from the sensor units 35 toward the biological tissue to observe the biological tissue. In this process, the oscillators 24 constituting the sensor units 35 intermittently transmit the ultrasound and receive the reflection wave from the biological tissue at a timing when the ultrasound is not transmitted. That is, when the sensor units 35 emit the ultrasound to the biological tissue, the transmission and reception of the ultrasound are switched in each predetermined period. When the observation of the biological tissue is completed, the irradiation units 36 transmit the ultrasound toward the distal tip 18 of the guide wire 15 to vibrate the distal tip 18 and thereby crush the occlusion site of the biological tissue.

A second usage mode will be described. Like the first usage mode, the four ultrasound sensors 23 are divided into the sensor units 35 and the irradiation units 36. After the observation of the biological tissue is completed, the timing of voltage application to the plurality of oscillators 24 constituting the sensor units 35 is set differently from that set at the time of the observation. With this setting, the oscillators 24 of the sensor units 35 transmit the ultrasound toward the distal tip 18 of the guide wire 15. That is, the ultrasound sensors 23, which have performed the observation function as the sensor units 35, exert the function as the irradiation units 36 for vibrating the distal tip 18 after the observation.

In the second usage mode, the sensor units 35 have an observation mode in which the ultrasound is emitted toward the occlusion site of the biological tissue and a crushing mode in which the ultrasound is emitted toward the distal tip 18. In the observation mode, the sensor units 35 are controlled so as to switch between the transmission and reception of the ultrasound in each predetermined period. In the crushing mode, the sensor units 35 are controlled so as to continuously transmit the ultrasound for a period longer than the predetermined period during the observation mode. According to the second usage mode, after observing the biological tissue, the ultrasound is transmitted toward the distal tip 18 not only from the irradiation units 36 but also from the sensor units 35, allowing the distal tip 18 to strongly vibrate. In the crushing mode in which the sensor units 35 emits the ultrasound toward the distal tip 18, the ultrasound is continuously transmitted. In this manner, the distal tip 18 can be effectively vibrated.

A third usage mode will be described. Like the first and second usage modes, the four ultrasound sensors 23 are divided into the sensor units 35 and the irradiation units 36 When the biological tissue is observed, the timing of voltage application to the plurality of oscillators 24 constituting the irradiation units 36 is set differently from that set at the time of the crushing. With this setting, the oscillators 24 of the irradiation units 36 transmit the ultrasound toward the biological tissue instead of the distal tip 18. That is, the ultrasound sensors 23, which are supposed to perform the crushing function as the irradiation units 36, exert the function as the sensor units 35 for observing the biological tissue before crushing the occlusion site.

According to such a usage mode, the biological tissue can be observed not only by the sensor units 35 but also by the irradiation units 36 prior to the passage through the occlusion site of the biological tissue. In this usage mode, the number of the ultrasound transmission position and reception position is increased as compared with the case where the observation is performed only by the sensor units 35. As a result, the observation accuracy of the shape of the biological tissue is improved. In the observation mode in which the irradiation units 36 transmit and receive the ultrasound, the ultrasound is transmitted and received intermittently and alternately. In this manner, the observation of the biological tissue can be surely performed by the irradiation units 36.

### <Fourth embodiment>

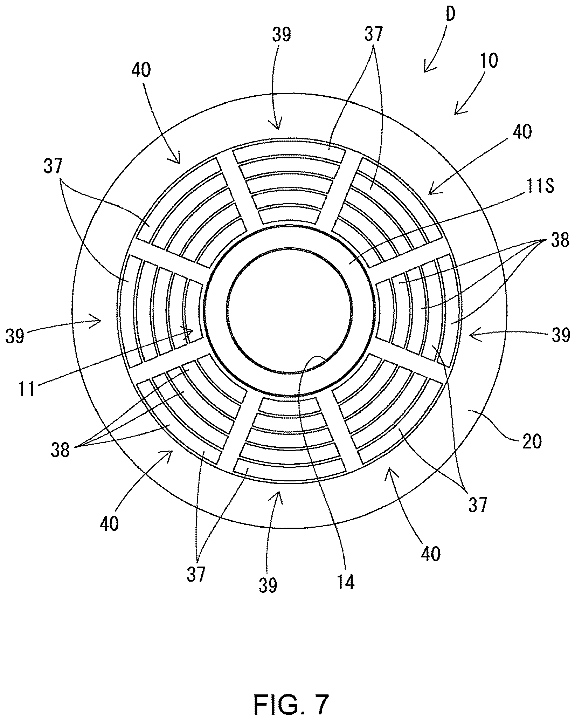

Next, a fourth embodiment embodying the disclosed embodiments will be described with reference to FIG. 7. In a catheter D of the fourth embodiment, a sensor unit 39 and an irradiation unit 40 have a configuration different from that in the above-mentioned first embodiment. Other configurations are the same as those in the above-mentioned first embodiment, thus the same configurations are designated by the same reference numerals, and description of the structure, functions, and effects thereof is omitted.

In the fourth embodiment, eight ultrasound sensors 37 are disposed to be aligned at an equal angular pitch in the circumferential direction around the lumen 14. Each ultrasound sensor 37 has a configuration in which a plurality of oscillators 38 are arranged in the radial direction. The plurality of oscillators 38 constituting each ultrasound sensor 37 are all formed in an arc shape. The peripheral length of the plurality of oscillators 38 gradually increases from the center side to the outer peripheral side. The ultrasound sensors 37 adjacent to each other in the circumferential direction are disposed side by side with a slit-like gap in the radial direction.

All of the oscillators 38 constituting the ultrasound sensors 37 are composed of piezoelectric elements. Thus, all of the ultrasound sensors 37 have both a function as the sensor units 39 for observing the biological tissue and a function as the irradiation units 40 for crushing the occlusion site of the biological tissue. In the fourth embodiment, the eight ultrasound sensors 37 are divided into the dedicated sensor units 39 and the dedicated irradiation units 40. In the fourth embodiment, the sensor units 39 and the irradiation units 40 are disposed so as to be arranged alternately in the circumferential direction. However, the arrangement order of the sensor units 39 and the irradiation units 40 can be freely set. Further, a modification of the fourth embodiment can have a configuration in which the eight ultrasound sensors 37 function as both the sensor units 39 and the irradiation units 40.

### <Fifth embodiment>

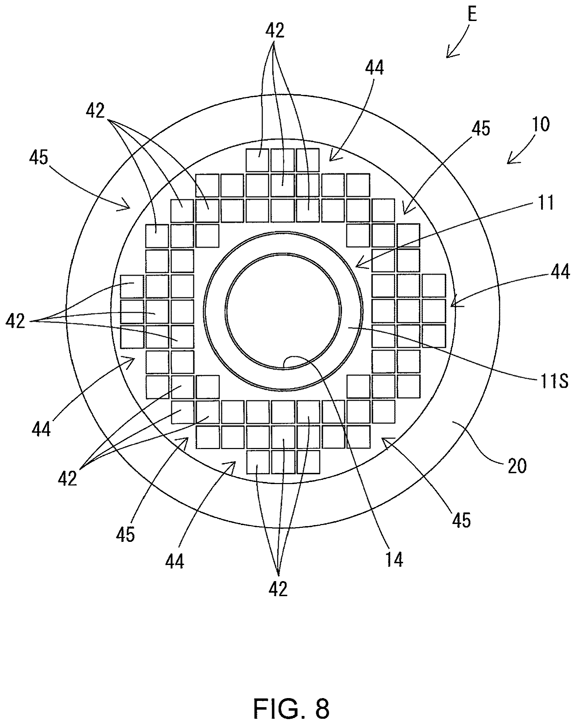

Next, a fifth embodiment embodying the disclosed embodiments will be described with reference to FIG. 8. In a catheter E of the fifth embodiment, a sensor unit 44 and an irradiation unit 45 have a configuration different from that in the above-mentioned first embodiment. Other configurations are the same as those in the above-mentioned first embodiment, thus the same configurations are designated by the same reference numerals, and description of the structure, functions, and effects thereof is omitted.

In the catheter E of the fifth embodiment, a plurality of oscillators 42 having a square shape are disposed so as to be aligned vertically and horizontally in an elevation view. The plurality of oscillators 42 are disposed in an annular region from the outer circumference of the lumen 14 to the inner circumference of the outer tube 20. Thus, all of the oscillators 42 can exert a function as the sensor unit 44 for observing the biological tissue and a function as the irradiation unit 45 for crushing the occlusion site of the biological tissue.

The sensor unit 44 is configured by one oscillator 42 or a plurality of oscillators 42. The irradiation unit 45 is also configured by one oscillator 42 or a plurality of oscillators 42. That is, the plurality of oscillators 42 are divided into the dedicated oscillator 42 constituting only the sensor units 44 and the dedicated oscillator 42 constituting only the irradiation units 45. In the fifth embodiment, the plurality of sensor units 44 and the plurality of irradiation units 45 are disposed so as to be arranged alternately in the circumferential direction. However, the arrangement order of the sensor units 44 and the irradiation unit 45 can be freely determined. Note that, a modification of the fifth embodiment can have a configuration in which all of the plurality of oscillators 42 can function as both the sensor units 44 and the irradiation units 45.

### <Other embodiments>

The disclosed embodiments are not limited to the embodiments described in the above description and drawings. For example, the following embodiments are also included in the technical scope of the disclosed embodiments.

In the above-mentioned first embodiment, the paired sensor units are disposed so as to sandwich the medical device. However, the paired sensor units may be disposed in a positional relationship not sandwiching the medical device.

In the above-mentioned first and second embodiments, the paired irradiation units are disposed so as to sandwich the medical device. However, the paired irradiation units may be disposed in a positional relationship not sandwiching the medical device.

In the above-mentioned first and second embodiments, two irradiation units are provided. However, the number of the irradiation units may be only one or three or more.

In the above-mentioned first to fifth embodiments, the oscillators constituting the sensor units and the oscillators constituting the irradiation units are composed of the same kind of component. However, the oscillators constituting the sensor units and the oscillators constituting the irradiation units may be different kinds of oscillators.

In the above-mentioned first to third embodiments, four ultrasound sensors are provided. However, the number of the ultrasound sensors may be three or less, or five or more.

The above-mentioned fifth embodiment shows the example in which the plurality of oscillators are disposed on the cross section of the distal end of the catheter. However, a configuration may be made such that a layer in which the plurality of oscillators are disposed is provided inside the cross section of the catheter, the oscillators constituting the irradiation units emit the ultrasound toward the medical device disposed beside the long axis of the catheter, and the oscillators constituting the sensor units observe the position of the medical device and the biological tissue in that direction.

### DESCRIPTION OF REFERENCE NUMERALS

- A, B, C, D, E: Catheter
- 10: Tube
- 14: Lumen
- 15: Guide wire (medical device)
- 24, 38, 42: Oscillator
- 25, 30, 35, 39, 44: Sensor unit
- 26, 36, 40, 45: Irradiation unit
- 27A: First transmission unit
- 27B: Second transmission unit

## Claims

1. A catheter comprising:
a tube having a lumen;
a sensor unit disposed at a distal end of the tube and capable of transmitting and receiving ultrasound; and
an irradiation unit disposed at the distal end of the tube and capable of emitting the ultrasound toward a medical device that passes through the lumen and protrudes from the distal end of the tube.

2. The catheter according to claim 1, wherein:
the irradiation unit is configured by arranging a plurality of oscillators in a row; and
the medical device is disposed on a line extended in an arrangement direction of the plurality of oscillators.

3. The catheter according to claim 2, wherein:
the irradiation unit includes a first transmission unit and a second transmission unit disposed so as to sandwich the lumen; and
the plurality of oscillators constituting the first transmission unit and the second transmission unit are disposed to be aligned on a straight line.

4. The catheter according to any one of claims 1 to 3, wherein the irradiation unit is configured by including oscillators that can be controlled independently of oscillators constituting the sensor unit.

5. The catheter according to any one of claims 1 to 4, wherein oscillators constituting the sensor unit and oscillators constituting the irradiation unit are composed of a same kind of component.

6. The catheter according to any one of claims 1 to 5, wherein the sensor unit is configured by including oscillators capable of transmitting the ultrasound toward the medical device.

7. The catheter according to claim 6, wherein the sensor unit has a mode of emitting the ultrasound toward a lesion and a mode of emitting the ultrasound toward the medical device, wherein:
in the mode of emitting the ultrasound toward the lesion, the sensor unit is controlled to switch between transmission and reception of the ultrasound in each predetermined period; and
in the mode of emitting the ultrasound toward the medical device, the sensor unit is controlled to continuously transmit the ultrasound for a period longer than the predetermined period.

8. The catheter according to any one of claims 1 to 7, wherein the irradiation unit is configured by including the oscillators capable of transmitting and receiving the ultrasound.

9. The catheter according to claim 8, wherein, in a mode of transmitting and receiving the ultrasound, the irradiation unit performs the transmission and reception of the ultrasound intermittently and alternately.

10. A catheter system comprising:
an emission control unit that is connected to the catheter according to any one of claims 1 to 9 and that controls ultrasound transmitting of the sensor unit and the irradiation unit, wherein
the emission control unit controls display on a display device on a basis of the ultrasound received by the sensor unit.
